# EUROPEAN PATENT APPLICATION

(11) **EP 2 962 696 A1**
(43) Date of publication of application: **06.01.2016**
(21) Application number: 14382250.0
(22) Date of filing: 30.06.2014
(51) Int. Cl.: A61K 39/102, C07K 14/285, C12N 1/36

(54) **Novel veterinary vaccine compositions for use in the treatment of infectious diseases caused by or associated with Pasteurellaceae family bacteria**

(71) Applicant: Aquilón Cyl S.L., 24004 León (ES); Universitat Autònoma de Barcelona, 08193 Bellaterra (ES)
(72) Inventor: Barbé Garcia, Jordi, 08290 Cerfanyola del Vallès (ES); Campoy Sánchez, Susana, 08221 Terrassa (ES); Llagostera Casas, Montserrat, 08290 Cerdanyola del Vallès (ES)
(74) Representative: Carlos Hernando, Borja

(57) **Abstract**

Novel veterinary vaccine compositions for use in the treatment of infectious diseases caused by or associated with *Pasteurellaceae* family bacteria.The present invention provides vaccine compositions comprising as active ingredient *Pasteurella multocida* strain PM1094 or an outer-membrane protein extract thereof for the therapeutic and prophylactic treatment in animals of respiratory infections which are caused by or associated with bacteria belonging to the family *Pasteurellaceae,* to improve animal health and productivity.

## Description

### FIELD OF THE INVENTION

The present invention relates to the field of vaccines, and their application as therapeutic and prophylactic agents in the veterinary industry, for the control of pathogen infections in livestock, caused by, or associated with bacteria belonging to the family *Pasteurellaceae.*

In particular, the present invention provides new vaccine compositions for preventing the onset of, and therapeutically treating, respiratory infections in animals which are caused by, or are associated with bacterial pathogens belonging to the family *Pasteurellaceae,* to improve animal health and productivity.

### BACKGROUND PRIOR ART

Respiratory diseases in livestock in general cause major welfare and economic problems for animal farms despite the widespread use of vaccines and antibiotics. Estimates of the financial cost run into millions per year, due to a combination of production losses, deaths, and veterinary bills.

Infectious respiratory disease in livestock is a major welfare and economic problem on many farms. Carrier animals that may show no sign of disease, but are able to infect others, are difficult to identify and treat. Even with modern drugs, bacteria and viruses in the upper respiratory tract are difficult to remove, making sick animals difficult to cure and susceptible to secondary infections.

Respiratory diseases suffered by farm animals are the results of a combination of primary and opportunistic infectious agents. In addition, adverse environmental and management conditions of the farms play an important role in the multifactorial nature of infectious respiratory diseases.

Primary pathogens are capable of subverting the animal own defense mechanisms and establish infection on their own, whereas, opportunistic pathogens, take advantage of the virulence mechanisms of the primary pathogens to establish infections. Bacterial agents may act as both primary and opportunistic pathogens, depending on the situation. Although primary respiratory infectious agents can cause serious disease on their own, more often uncomplicated infections with these agents are mild and transient. It is when these primary infections become complicated with opportunistic bacteria that more serious and chronic respiratory diseases result.

Most commonly isolated or studied bacterial pathogens, being the cause or contributing to the onset of a respiratory infectious disease in livestock animals are pathogens belonging to the family of *Pasteurellaceae* bacteria, such as for example, *Pasteurella sp., Mannheimia sp., Haemophilius sp., Histophilus sp.,* and *Actinobacillus sp.*

Porcine pleuropneumonia is one of the most important respiratory diseases of intensively raised swine throughout the world. It is caused by the gram-negative bacterium *Actinobacillus pleuropneumoniae.* The economic consequences of porcine pleuropneumoniae can be severe and are principally due to death, decrease average daily gain in weight and feed conversion ratios, slaughter trim losses and intervention costs. Pigs of all ages may be affected, but those 3 to 4 month of age are most frequently found to have this infection. The clinical course of porcine pleuropneumonia can be peracute and acute (characterized by dyspnea, cough, apathy, anorexia and sudden death) or subacute and chronic (characterized by intermittent cough and decrease growth parameters.

*Haemophilus parasuis* is a member of the family *Pasteurellaceae* and a common inhabitant of the upper respiratory tract of healthy pigs. It is also known as the etiological agent of Glässer's disease in swine, a systemic disease characterized by fibrinous polyserosytis, which causes high morbidity and mortality in piglets. *H. parasuis* can also produce pneumonia and sudden death. Glässer's disease has gained considerable importance in recent years and it is recognized as one of the main causes of economic loss in the pig industry.

Pneumonic pasteurellosis is one of the most economically important infectious diseases of ruminants with a wide prevalence throughout the continents. The disease is characterized by an acute febrile course with severe fibrinous or fibrinopurulent bronchopneumonia, fibrinous pleurisy and septicaemia. Infected animals may die within a few days of the onset of clinical signs, but those which survive the acute attack may become chronically infected. *Mannheimia haemolytica* is well established to be the major aetiological agent of the disease although *Pasteurella multocida* has also been incriminated in many acute outbreaks. Both *Mannheimia* and *Pasteurella* species are commensally resident in the respiratory tract of healthy ruminants and are capable of causing infection in animals with compromised pulmonary defense system. Hence, the disease is essentially triggered by physical or physiological stress created by adverse environmental and climatic conditions such as extremely bad weather, poor management, overcrowding, transportation or previous infection with respiratory viruses, mycoplasma or some other pathogenic organisms.

It is worth mentioning that *Mannheimia haemolytica, Pasteurella multocida* and *Pasteurella trehalosi* constitute the most important members of the family *Pasteurellaceae* that pose serious hazards in livestock industry. These species are commensally resident in the animal body as normal constituents of the nasopharyngeal microflora and are all capable of causing infection when the body defense mechanisms are impaired. Their presence is mainly confined to ruminants with most adequately characterized strains originating from cattle, sheep and goats (Biberstein & Hirsh, 1999). Examples of the most commonly recognized diseases associated with *Mannheimia haemolytica* include shipping fever in cattle, primary and secondary pneumonia in cattle sheep and goats, septicaemia and mastitis in sheep and a number of non-specific inflammatory lesions in various species of domestic animals (Quinn et al., 2002). *Pasteurella multocida* is, on the other hand, associated with haemorrhagic septicaemia in cattle and buffaloes and enzootic pneumonia complex in young ruminants (Jones et al.,1997). Other diseases such as fowl cholera and snuffles (an upper respiratory tract infection occasionally accompanied by pleurisy, pneumonia or fatal septicaemia in rabbits) are also caused by *Pasteurella multocida.* With regard to *Pasteurella trehalosi* this organism is frequently associated with acute systemic disease or septicaemia in young sheep (Dyson et al., 1981; Jones et al., 1997).

In particular, *Mannheimia haemolytica* is the principal bacterium isolated from respiratory disease in feedlot cattle and is a significant component of enzootic pneumonia in all neonatal calves. For example, in the United States, *Mannheimia haemolytica,* serotype 1 is the main bacteria responsible for the clinical signs and lesions of severe bovine pneumonia (shipping fever), particularly disease that is seen within the first week to 10 days after stress such as shipping.

The bacterium is a normal inhabitant of nasal passages and tonsils of ruminants. Following stress or viral infections, the bacterium proliferates and is inhaled into the lungs where it stimulates acute signs of severe respiratory distress: coughing, nasal discharge, high fever, loss of appetite and, maybe, death. The typical lesion is inflammation of the lung (pneumonia) and chest cavity (pleuritis). These lesions if inadequately treated, can become co-infected with other bacteria, including *Mycoplasma,* resulting in chronic pneumonia with scarring and abscess formation.

*Histophilus somni* (formerly *Haemophilus somnus)* is a Gram-negative bacterium that is a member of the *Pasteurellaceae* family. It appears microscopically as a cocco-bacillus, is a facultative anaerobe, non-motile, and a non-spore-forming bacteria. *Histophilus somni* is a commensal organism of cattle that may inhabit certain mucosal surfaces, including the upper airway and reproductive tract. *Histophilus somni* was first recognized as a pathogen in cattle in 1956. *Histophilus somni* is a pathogen of cattle worldwide, but the greatest prevalence of disease is focused in the large beef producing countries of the world, such as the United States and Canada. Infection with *Histophilus somni* is most commonly a feedlot disease, but may be seen in dairy and grazing operations. Young growing cattle age 6-12 months are most commonly infected and show clinical signs. The actual prevalence of the bacteria is very high, and almost all cattle will be exposed at some point in their life. This can be noted in certain herds where 100% of animals have circulating antibodies to *Histophilus somni.* Actual clinical disease, however, is uncommon in susceptible groups, with an incidence rate of 1-2% lower. Clinical disease can be devastating when it occurs. *Histophilus somni* is capable of causing a variety of disease syndromes, including thrombotic meningoencephalitis (TME), respiratory disease *(Histophilus somni* is a component of the Bovine Respiratory Disease Complex, BRDC), myocarditis, polysynovitis, otitis media, mastitis, and reproductive tract diseases. Several different approaches have been used so far in the treatment of respiratory diseases caused by bacterial pathogens in livestock, however there is still a need for further technical improvement in the present field, to increase efficiency of therapeutic treatments and prevention of the diseases.

The state of the art describes treatment methods with antibiotics and vaccines. However, providing an effective agent or treatment method is complicated, as isolating and detecting the primary and/or relevant opportunistic pathogens in an infectious respiratory disease is not always possible.

Moreover, existing treatment methods are based on therapeutic agents or vaccines directed to a specific pathogen, which although effective against that specific pathogen, do not provide the desired preventive or therapeutic effect when treating an infectious respiratory disease, caused or associated with different pathogens.

The present invention is therefore directed to provide vaccine compositions with improved effectiveness for the prevention and therapeutic treatment of infectious respiratory disease in livestock animals caused by, or being associated with bacterial pathogens belonging to the family of *Pasteurellaceae* bacteria.

### SUMMARY OF THE INVENTION

The term "infectious respiratory diseases caused by, or being associated with bacterial pathogens" in the context of the present invention refers to any respiratory disease suffered by livestock animals, which etiology is the result of primary infection or opportunistic infection by at least a bacterial pathogen belonging to the family of *Pasteurellaceae* bacteria. Non limiting examples thereof include: acute, subacute or chronic pneumonia, shipping fever, pleuropneumonia, porcine respiratory disease complex, bovine respiratory disease complex, upper respiratory tract infections, diphteria, lower respiratory tract infections, Glässer's disease and pasteurellosis.

The terms "livestock animals or animals" in the context of the present invention means animals raised in an agricultural setting to produce commodities such as food, fiber and labor and include: cattle, horses, pigs, birds, poultry, sheep, goat and rabbit.

The term, "bacterial pathogen" in the context of the present invention refers to bacteria which cause a clinically evident illness (i.e., characteristic medical signs and/or symptoms of disease) resulting from a primary or secondary infection, presence and growth in a host animal.

The term "family of *Pasteurellaceae* bacteria" in the context of the present invention is meant to include a large family of gram-negative bacteria from the genera *Haemophilus, Pasteurella, Actinbacillus* and *Mannheimia,* but excluding any virulent strain and serotype of *Pasteurella multocida.* In particular, bacteria belonging to the family of *Pasteurellaceae* bacteria according to the present invention comprise any virulent strain and/or serotype of *Mannheimia sp., Haemophilius sp., Histophilus sp., Pasteurella sp.* (other than *Pasteurella multocida),* and *Actinobacillus sp..* In particular, bacteria species belonging to the *Pasteurellaceae* family according to the present invention are selected from the group consisting of: *Manheimia haemolytica, Haemophilus parasuis, Actinobacillus pleuropneumoniae, Histophilus somni, Pasteurella trehalosi* and *Haemophilus paragallinarum.*

The term "therapeutic treatment" in the context of the present invention encompasses preventing or reducing the severity of/or associated with the symptoms of an infectious respiratory disease in an animal (livestock) caused by a bacterial pathogen belonging to the *Pasteurellaceae* family.

The term "prophylactic treatment" in the context of the present invention encompasses the treatment of an animal (livestock) not suffering from an infectious respiratory disease caused by a bacterial pathogen belonging to the *Pasteurellaceae* family, to prevent or at least reduce the likelihood of that animal suffering from that infectious respiratory disease.

The term "acceptable carrier, vehicles or coadjuvants" in the context of the present invention are meant to include any carrier, whether pharmaceutically acceptable or not, which does not cause significant irritation to an organism and does not abrogate the biological activity and properties of the administered compound/composition. These vehicles include those acceptable and suitable for administering the compound/composition for an intended prophylactic and/or therapeutic treatment. Examples thereof include: lipopolysacchrarides, the Freund's complete or incomplete adjuvant, monophopholipids, such as, monophospholipid A, sulfates, phosphates such as aluminium phosphate, and hydroxides, such as hydrated aluminum hydroxyphosphate and aluminium hydroxide, etc.

According to a first aspect, the invention relates to *Pasteurella multocida* strain PM1094 or an outer-membrane protein extract prepared therefrom for use in the prophylactic and therapeutic treatment of respiratory infections in livestock animals caused by, or associated with at least one bacterial pathogen belonging to the *Pasteurellaceae* family other than any virulent strain and serotype of the bacterial species *Pasteurella multocida.*

According to a second aspect, the present invention relates to *Pasteurella multocida* strain PM1094 or an outer-membrane protein extract prepared therefrom for use as defined above, wherein the bacterial pathogen belonging to the *Pasteurellaceae* family is selected from the group consisting of: any virulent strain and/or serotype of *Mannheimia sp., Haemophilius sp., Histophilus sp., Pasteurella sp.* (other than *Pasteurella multocida*), and *Actinobacillus sp..*

According to a third aspect, the present invention relates to *Pasteurella multocida* strain PM1094 or an outer-membrane protein extract prepared therefrom for use as defined above, wherein the bacterial pathogen belonging to the *Pasteurellaceae* family is selected from the group consisting of: any virulent strain and/or serotype of *Manheimia haemolytica, Haemophilus parasuis, Actinobacillus pleuropneumoniae, Histophilus somni, Pasteurella trehalosi* and *Haemophilus paragallinarum.*

According to a further aspect the invention relates to *Pasteurella multocida* strain PM1094 or an outer-membrane protein extract prepared therefrom for use as defined previously, wherein the respiratory infections caused by, or associated with at least one pathogen belonging to the *Pasteurellaceae* family are selected from the group consisting of: acute, subacute or chronic pneumonia, shipping fever, pleuropneumonia, porcine respiratory disease complex, bovine respiratory disease complex, upper respiratory tract infections, diphteria, lower respiratory tract infections, Grasser's disease and pasteurellosis.

A still further aspect of the invention relates to *Pasteurella multocida* strain PM1094 or an outer-membrane protein extract prepared therefrom for use as previously defined in the prophylactic and therapeutic treatment of respiratory infections in livestock animals selected from the group consisting of: cattle, horses, pigs, birds, poultry, sheep, goat and rabbit.

Additional objects of the present invention are *Pasteurella multocida* strain PM1094 or an outer-membrane protein extract prepared therefrom for use as previously defined in the prophylactic and therapeutic treatment of respiratory infections in pigs caused by or associated with *Actinobacillus pleuropneumoniae,* in the prophylactic and therapeutic treatment of respiratory infections in pig, cattle or sheep caused by or associated with *Manheimia haemolytica,* in the prophylactic and therapeutic treatment of respiratory infections in pigs caused by or associated with *Haemophilus parasuis,* in the prophylactic and therapeutic treatment of respiratory infections in cattle caused by or associated with *Histophilus somni,* in the prophylactic and therapeutic treatment of respiratory infections in sheep caused by or associated with *Pasteurella trehalosi,* and in the prophylactic and therapeutic treatment of respiratory infections in poultry caused by or associated with *Haemophilus paragallinarum.*

It is also an object of the present invention vaccine compositions against respiratory infections in livestock animals caused by, or associated with at least one bacterial pathogen belonging to the *Pasteurellaceae* family other than any virulent strain and serotype of the bacterial species *Pasteurella multocida,* as defined previously, comprising an immunogenically effective amount of *Pasteurella multocida* strain PM1094 or an outer-membrane protein extract prepared therefrom together with an acceptable carrier, vehicle and/or adjuvant.

In a further embodiment the invention provides a vaccine for inducing an immune response in an animal suffering from, or susceptible to, a condition associated with respiratory infections caused by or associated with at least one bacterial pathogen belonging to the *Pasteurellaceae* family, other than any virulent strain and serotype of *Pasteurella multocida,* which comprises as active ingredient an effective amount of *Pasteurella multocida* strain PM1094 or an outer-membrane protein extract prepared therefrom.

In a still further embodiment the invention provides a method of increasing survival of an animal suffering from a respiratory disease caused by or associated with at least one bacterial pathogen belonging to the *Pasteurellaceae* family, other than any virulent strain and serotype of *Pasteurella multocida,* said method comprising administering to the infected animal an effective amount of *Pasteurella multocida* strain PM1094 or an outer-membrane protein extract prepared therefrom together with suitable carriers and vehicles.

### FIGURES

**Figure 1****:** Specific immunoglobulin against OMPs of several strains of *Haemophilius parasuis, Haemophilus influenza* and *Manhemia haemolytica.*
**Figure 2****:** Specific immunoglobulin against OMPs of several strains of *Actinobacillus pleuropneumoniae.*
**Figure 3****:** Specific immunoglobulin against OMPs of several strains of *Haemophilius parasuis, Haemophilus influenza* and *Manhemia haemolytica.after* subcutaneous vaccination.
**Figure 4****:** Specific immunoglobulin against OMPs of several strains of *Haemophilius parasuis, Haemophilus influenza* and *Manhemia haemolytica.after* intraperitoneal vaccination.
**Figure 6****:** Specific immunoglobulin against OMPs of several strains of *Actinobacillus pleuropneumoniae* after intraperitoneal vaccination.
**Figure 7****:** Quantification of specific IgG against OMP of *Pasteurella multocida* PM1094 (the vaccine strain) (A), and *Actinobacillus pleuropneumoniae* (B) strains used for the challenge. Day 0, corresponds to the data of sera collected on day 0 of all animal groups; Day 35 are the data of sera collected on day 35 for all the vaccinated (T3 group) and non-vaccinated (T4 group) animals. No differences are detected between Day 0 and Day 35 non-vaccinated. The statistical significances are p<0.01 (***) in one-way ANOVA analysis.
**Figure 8****:** Lung scores (average ± standard deviation) from all the animals from groups T03 (vaccinated) and T04 (non-vaccinated), after infection with *A. pleuropneumoniae.*
**Figure 9****:** Lung scores (average ± standard deviation) from animals with lesions from groups T03 (vaccinated) and T04 (non-vaccinated), after infection with *A. pleuropneumoniae.*
**Figure 10****:** Dyspnea observed in the animals from group T03 (vaccinated) and T04 (non-vaccinated) after infection with *A. pleuropneumoniae.* Upper panel: percentage of animals affected; lower panel: mean dyspnea scores±standard deviation.
**Figure 11****:** Coughing observed in the animals from group T03 (vaccinated) and T04 (non-vaccinated) after infection with *A. pleuropneumoniae.* Upper panel: percentage of animals affected; lower panel: mean cough scores±standard deviation.
**Figure 12****:** Sneezing observed in the animals from group T03 (vaccinated) and T04 (non-vaccinated) after infection with *A. pleuropneumoniae.* Upper panel: percentage of animals affected; lower panel: mean sneezing scores±standard deviation.
**Figure 13****:** Body temperature of the pigs infected with *Actinobacillus pleuropneumoniae.* Data are from vaccinated animals (group T03) and non-vaccinated animals (group T04).
**Figure 14****:** Survival of vaccinated (T03) and non-vaccinated (T04) animals after A. *pleuropneumoniae* infection.

### DETAILED DESCRIPTION OF THE INVENTION

The present invention provides novel vaccine formulations for the therapeutic and prophylactic treatment of respiratory infectious diseases in livestock animals which are caused by, or are associated with bacterial pathogens belonging to the *Pasteurellaceae* family, excluding any virulent strain and serotype of the species *Pasteurella multocida.*

The inventors of the present application have surprisingly found that the already known mutant *Pasteurella multocida subsp. multocida* strain CECT 8081 (PM1094), described in international patent application WO 2009/095518, unexpectedly confers protection in animals against bacterial pathogens belonging to the *Pasteurellaceae* family, and are therefore to be used according to the present invention in vaccine formulations for use in the treatment and prevention of respiratory infectious diseases caused in animals (livestock) by bacterial pathogens belonging to the *Pasteurellaceae* family.

As will be shown in the present invention, vaccination with *Pasteurella multocida* strain PM1094 is effective in eliciting antibodies against the outer membrane proteins (OMP) of bacterial pathogens belonging to the *Pasteurellaceae* family, is also effective in reducing the clinical signs associated to respiratory infections, such as lung scores, dyspnea, coughing, sneezing and body temperature and also reduces the mortality rate of infected animals.

Iron uptake is essential for *in vivo* survival of bacterial pathogens, since free iron inside the host is very limited. This function is regulated by the repressor Fur, which is active under iron rich conditions (these are the conditions commonly found in laboratory culture media). Therefore, in the laboratory, bacteria do not need special systems for iron uptake and these systems remain repressed under the action of Fur. However, during infection, these iron-uptake systems are expressed to guarantee the survival of the pathogen inside the host. For the purpose of the present invention, the *in vivo* situation was mimic by limiting the quantity of free iron in the culture medium (under these conditions bacterial growth is reduced) or by elimination of Fur by mutation. Mutations of the *fur* gene in bacteria produce a derepression of genes involved in iron acquisition. Thus, the elimination of the repression action of Fur translates in an increased transcription and subsequent translation of iron-uptake proteins, some of which are located on the bacterial surface. This is the case for *Pasteurella multocida* and a bacterine produced with a *fur* mutant has enhanced protective capacity. As has been surprisingly found in the present invention, the mutant *Pasteurella multocida* strain PM1094 and outer membrane proteins (OMPs) thereof showed cross-reaction with OMPs from other bacterial species from the *Pasteurellaceae* family, including *Actinobacillus pleuropneumoniae.* This cross-reactivity supports the possibility of providing protection with the *Pasteurella multocida* strain 1094 against pathogens belonging to the *Pasteurellaceae* family as defined above.

The strain PM1094 is deposited in the Colección Española de Cultivos Tipo (Spanish Collection of Type Cultures), Parc Cientific Universitat de Valencia, Catedratico Agustin Escardino, 9, 46980 Paterna (Valencia), Spain, with the registration number CECT 8180 deposited on October 1, 2012, according to the Treaty of Budapest of 28^{th} April 1977.

The deposited mutant *P.multocida* strain CECT 8081 (PM1094) is a double mutant, defective in the Fur and OmpH proteins.

The method for obtaining strain PM1094 is disclosed in WO 2009/095518 and contemplates mutation of the *fur* gene which consists in disruption of the gene by introducing in the bacteria a plasmid containing a fragment of 394 base pairs of the internal region of this gene comprised between nucleotides 18 and 412, and carrying out the nonsense mutations in the *ompH* genes (*ompH1* and *ompH2* genes). The mutation in *ompH1* is a nonsense mutation in position 76 which gives rise to a stop codon instead of a glutamine codon, making it express a truncated protein with 24 amino acids. The mutation in *ompH2* involves several nucleotide changes, including a nonsense mutation in position 670, which gives rise to a truncated protein with 223 amino acids instead of the 350 which the native protein has. The effect caused by the nonsense mutation in *ompH1* and *ompH2* is the absence of the 36KDa outer-membrane protein.

Strain PM1094 is known from WO 2009/095518 to provide heterologous protection against virulent *Pasteurella multocida.* This patent application describes compositions and vaccines containing the said strain (inactivated) or an outer-membrane protein extract prepared there from to be used as immunogenic agents for the protection against virulent *Pasteurella multocida* in pigs, cattle and small mammals.

The vaccine formulations according to the present invention comprise the mutant *Pasteurella multocida* strain PM1094 containing the previously described mutations, which are inactivated thermally or by sonication, or an outer-membrane protein extract prepared therefrom, together with suitable carrier and vehicles.

Inactivation of the strain PM1094 is carried out by thermal treatment at 45-55°C, more preferably at 50°C, during at least 16 h.

Outer-membrane separation techniques used in accordance with the present invention involved the suspension of cells at a concentration from 10¹⁰ to 10¹² CFU/ml, preferably 10¹¹ CFU/ml, in acetate buffer (0.2 M sodium acetate and 0.2 M lithium chloride), cell disruption with a syringe of 19 gauges and centrifugation at 10 000 rpm during 15 min at 4°C. Afterwards, the supernatant was collected and centrifuged at 23 000 rpm during 2 h 30 min at 4°C. Finally, the pellet obtained was resuspended in ultrapure water.

To formulate the vaccine of the present invention, the inactivated strain PM1094 or an outer-membrane protein extract prepared therefrom, is combined according to standard practices with any of the typical carrier, vehicles or coadjuvants in veterinary vaccine practice, such as, lipopolysacchrarides, the Freund's complete or incomplete adjuvant, monophopholipids, such as, monophospholipid A, sulfates, phosphates such as aluminium phosphate, and hydroxides, such as hydrated aluminum hydroxyphosphate and aluminium hydroxide.

The dose of the vaccine will vary depending on the concentration of the antigenic material, ranging from 10⁶ to 10¹⁰ CFU/animal, more preferably, from 10⁷ to 10⁹ CFU/animal. Possible vaccination routes are: intramuscular, oral, intraperitoneal, intratranasal, intratracheal, subcutaneous, etc. Preferred route is intramuscular or intraperitoneal.

Experimental evidence of the invention's industrial application as well as the advantages provided by the invention are detailed in the examples of the present specification.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are intended for purposes of illustration only and are not intended to limit the scope of protection. Furthermore, the present invention covers all possible combinations of particular and preferred embodiments described herein.

### EXAMPLES

### Example 1: Evaluation in mice of the protection against a heterologous challenge (240xLD₅₀), with or without adjuvant

To determine the protective effect of the *Pasteurella multocida* PM1094 strain, four groups of six three-weeks old female Hsd:ICR(CD-1) mice were used. One group was treated with PM1094 inactivated cells at a dose of 10⁸ CFU/animal without adjuvant. Likewise, the second one was also vaccinated but aluminum hydroxide (33% v/v) adjuvant was added to the vaccine. The third and fourth groups were the non-vaccinated controls with and without adjuvant. The vaccine was administrated at days 1 and 15 of the experiment. Challenge of all the groups was performed at a dose of 240 x LD50 by intraperitoneal inoculation with the *Pasteurella multocida* PM25 strain which is of a different serovar than that of the vaccine strain. Animals were daily supervised, death was recorded and the percentage of survival was calculated. Results found indicated that vaccine protects the 100% of animals, because all vaccinated animals survived to the challenge.

**Table 1. Protection conferred by the vaccine against a heterologous challenge**

| Group | Adjuvant | Number of animals | Number of dead animals | % Protection |
|---|---|---|---|---|
| Negative control | - | 6 | 6 | 0 |
| Adjuvant control | + | 6 | 6 | 0 |
| Group 1 | - | 6 | 0 | 100 |
| Group 2 | + | 6 | 0 | 100 |

### Example 2: Evaluation in mice of the immune response against OMPS of, Haemophilius parasuis, Haemophilus influenzae, Manhemia haemolytica, and Actinobacillus pleuropneumoniae.

To evaluate the mice immune response after vaccination, ELISA assays were performed to determine the presence, in mouse sera, of specific immunoglobulin (Ig) against OMPs of several bacterial strains (*Haemophilius parasuis* C196 and Nagasaki strains, *Haemophilus influenzae, Manhemia haemolytica* PH219, and PH214 strains, *Actinobacillus pleuropneumoniae* C140, CV600, CV424, CV601 and CV-411 strains and *Salmonella enterica* LT2 strain).

To obtain the serum samples, four groups of six three-weeks old female Hsd:ICR(CD-1) mice were used. One group was treated intraperitoneally with PM1094 inactivated cells at a dose of 10⁸ CFU/animal without adjuvant. Likewise, the second one was also vaccinated but adjuvant aluminum hydroxide (33% v/v) adjuvant was added to the vaccine. The third and fourth groups were the non-vaccinated controls with and without adjuvant. The vaccine was administrated at days 1 and 15 of the experiment. At days 1 (before the first inoculation) and 30, three animals of each group were euthanized and sera were obtained.

For the ELISA assay, OMPs of the different pathogens were obtained. To do this, cells at a concentration of 10¹¹cfu/ml were resuspended in acetate buffer (0.2 M sodium acetate and 0.2 M lithium chloride), cell disruption was performed by using a syringe of 19 gauges. Then, the suspension was centrifuged at 10 000 rpm during 15 min at 4°C, the supernatant was collected and centrifuged at 23 000 rpm during 2 h 30 min at 4°C. Finally, the pellet obtained was resuspended in ultrapure water.

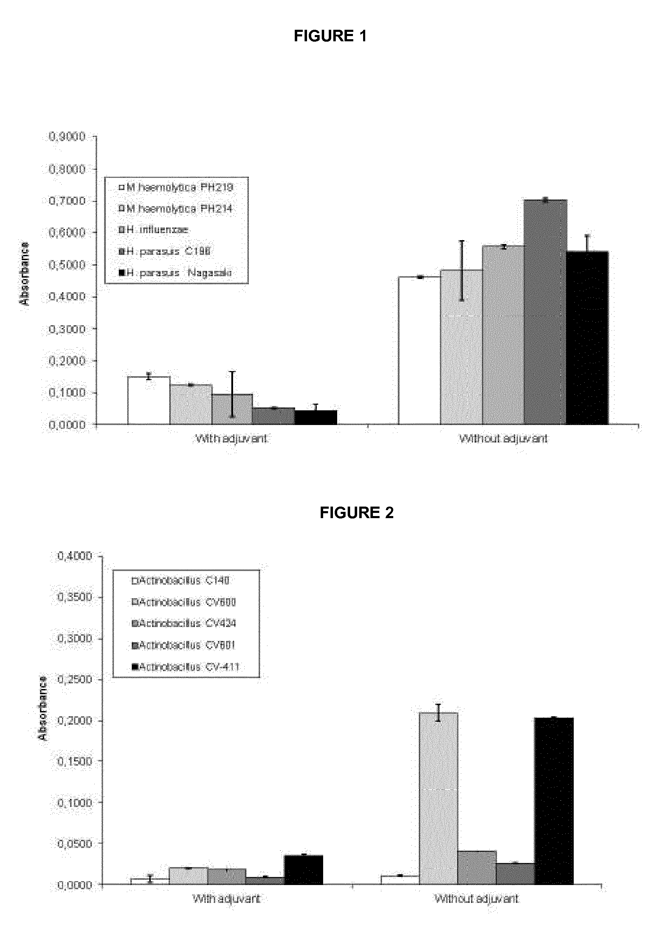

For each bacterial strain, the corresponding OMPs (0.5 µg per well) were immobilized in microtiter plates. ELISA assays were performed using suitable mice sera dilution and an anti-mice Ig HRP-conjugated antibody as secondary antibody. The obtained results indicate that all animals treated with the inactivated PM1094 vaccine (with or without adjuvant) present a significant increase of the specific Ig against OMPs of all bacteria assayed except for those of *Salmonella enterica* strain, which was used as a negative control due to its phylogenetic distance with the *Pasteurellaceae* family. See Figures 1 and 2.

### Example 3: Evaluation in rabbit of the immune response against OMP from strains of M. haemolytica, H.parasuis and H. influenza

To evaluate the rabbit immune response after vaccination, ELISA assays were performed to determine the presence, in rabbit sera, of specific immunoglobulin (Ig) against OMPs of several bacterial strains (*Haemophilius parasuis* C196 and Nagasaki strains, *Haemophilus influenzae, Manhemia haemolytica* PH219, and PH214 strains and *Salmonella enterica* LT2 strain).

To obtain the serum samples, four groups of six rabbits each one were used. One group was treated with PM1094 inactivated cells at a dose of 10¹⁰ CFU/animal by subcutaneous innoculation. Likewise, the second one was also vaccinated but by intraperitoneal administration. The third and fourth groups were the non-vaccinated controls treated with saline a suspension by subcutaneous or intraperitoneal injection. The vaccine was administrated at days 1 and 15 of the experiment. At days 1 (before the first inoculation) and 30, serum samples were collected for all the animals of each group.

For the ELISA assay, OMPs of the different pathogens were obtained. To do this, cells at a concentration of 10¹¹cfu/ml were resuspended in acetate buffer (0.2 M sodium acetate and 0.2 M lithium chloride), cell disruption was performed by using a syringe of 19 gauges. Then, the suspension was centrifuged at 10 000 rpm during 15 min at 4°C, the supernatant was collected and centrifuged at 23 000 rpm during 2 h 30 min at 4°C. Finally, the pellet obtained was resuspended in ultrapure water.

For each bacterial strain, the corresponding OMPs (0.5 µg per well) were immobilized in microtiter plates. ELISA assays were performed using suitable mice sera dilution and an anti-rabbit Ig HRP-conjugated antibody as secondary antibody.

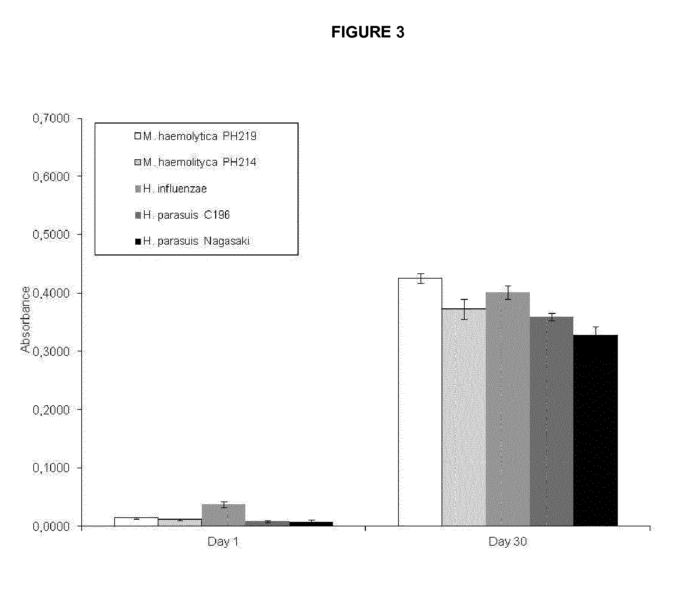

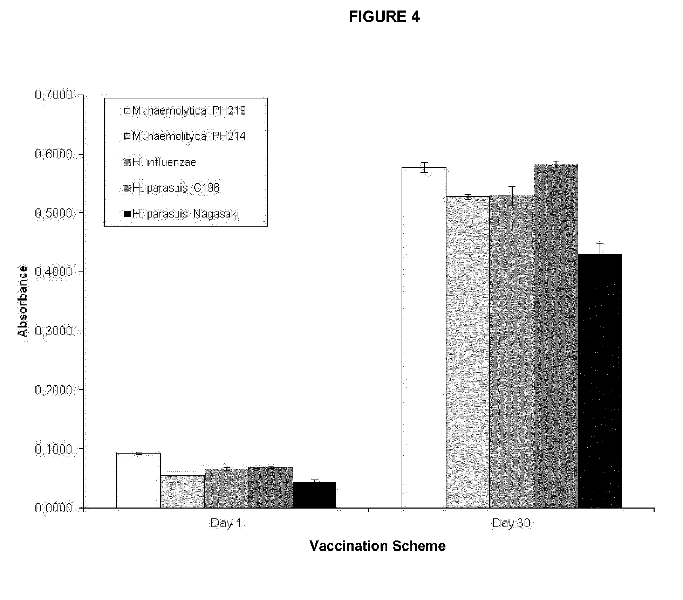

The obtained results indicate that all animals treated with the inactivated PM1094 vaccine (either by subcutaneous or intraperitoneal administration) present a significant increase of the specific Ig against OMPs of all bacteria assayed except for those of *Salmonella enterica* strain which was used as a negative control due to its phylogenetic distance with the *Pasteurellaceae* family. See Figures 3 and 4.

Subcutaneous and Intraperitoneal vaccination induce an increase in serum total Ig against OMP from strains of M. haemolytica, H.parasuis and H. influenza.

### Example 4: Evaluation in rabbit of the immune response against OMP from strains of Actinobacillus pleuropneumoniae

To evaluate the rabbit immune response after PM1094 vaccination against OMPs of *Actinobacillus pleuropneumoniae,* ELISA assays were performed using OMPs of *A. pleuropneumoniae* C140, CV600, CV424, CV601 and CV-411 strains and S. *enterica* LT2 strain.

For serum samples obtaining, four groups of six rabbits each one were used. One group was treated with PM1094 inactivated cells at a dose of 10¹⁰ CFU/animal by subcutaneous inoculation. Likewise, the second one was also vaccinated but by intraperitoneal administration. The third and fourth groups were the non-vaccinated controls treated with saline a suspension by subcutaneous or intraperitoneal injection. The vaccine was administrated at days 1 and 15 of the experiment. At days 1 (before the first inoculation) and 30, serum samples were collected for all the animals of each group.

For the ELISA assay, OMPs of S. *enterica* LT2 and of the different *A. pleuropneumoniae* strains (C140, CV600, CV424, CV601 and CV-411) were obtained. To do this, cells at a concentration of 10¹¹cfu/ml were resuspended in acetate buffer (0.2 M sodium acetate and 0.2 M lithium chloride), cell disruption was performed by using a syringe of 19 gauges. Then, the suspension was centrifuged at 10 000 rpm during 15 min at 4°C, the supernatant was collected and centrifuged at 23 000 rpm during 2 h 30 min at 4°C. Finally, the pellet obtained was resuspended in ultrapure water.

For each bacterial strain, the corresponding OMPs (0.5 µg per well) were immobilized in microtiter plates. ELISA assays were performed using suitable mice sera dilution and an anti-rabbit Ig HRP-conjugated antibody as secondary antibody.

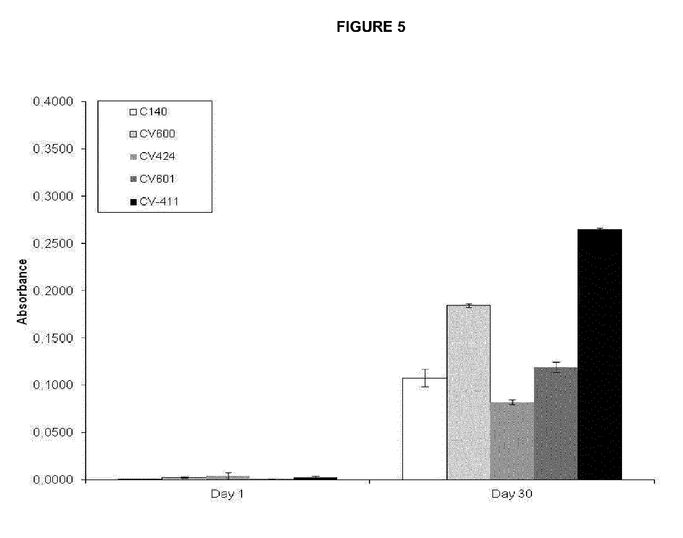

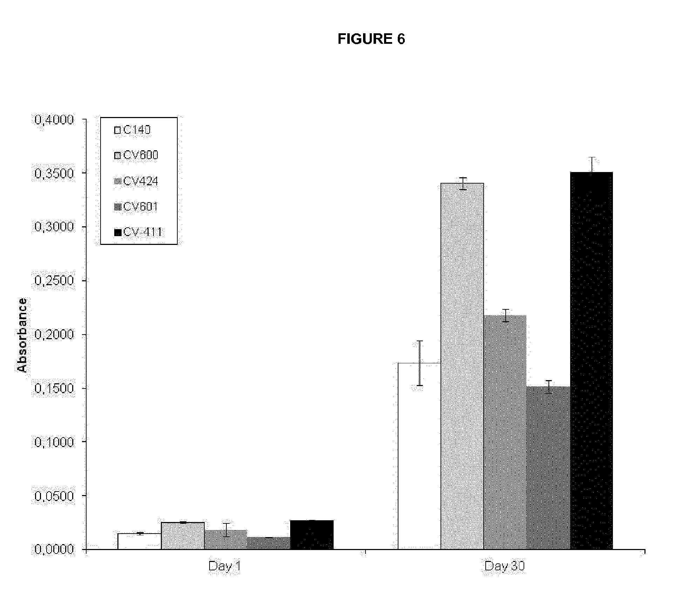

The obtained results indicate that all animals treated with the inactivated PM1094 vaccine (either by subcutaneous or intraperitoneal administration) present a significant increase of the specific Ig against OMPs of all *A. pleuropneumoniae* strains assayed. As expected, no cross reaction exist when OMPs of *S.enterica* LT2 strain were used due to its phylogenetic distance with *Pasteurellaceae* family. See Figures 5 and 6.

Subcutaneous and Intraperitoneal vaccination induce an increase in serum total Ig against OMP from strains of Actinobacillus pleuropneumoniae.

### Example 5: Obtaining a vaccine preparation based in PM1094

Strain PM1094 was grown overnight at 37°C on several sheep-blood agar plates (COS, Biomerieux, Madrid, Spain). Once grown, the bacterial biomass was harvest and resuspended in buffered peptone water (BPW, Merck Darmstadt, Germany) generating a concentrated cell suspension stock at about 10¹¹ CFU/ml. The exact bacterial concentration was determined by serial dilution plating on sheep-blood agar plates. The suspension stock was then inactivated by heating at 50°C for 18h. The complete cell inactivation was confirmed by plating 200µl of the cell suspension. The inactivated vaccine was prepared by diluting the inactivated cell suspension stock to a final concentration of 1x10¹⁰ inactivated CFU/ml and by adding timerosal [1/1000 (v/v)] and aluminum hydroxide [1/3 (v/v)]. Again 1ml of the vaccine was plated onto sheep-blood agar plates to ensure that no contamination has occurred. The vaccine was stored at 4°C until used.

Titration. The concentration of the concentrated stock was 7.1 x 10¹⁰ CFU/ml. After inactivation, 15 ml of this concentrated stock was then mixed with 33 ml of aluminum hydroxide, 52 ml of BPW and 0.1 ml of timerosal. Then, the final concentration of the inactivated and adjuvanted vaccine was 1.06 x 10¹⁰ CFU/ml.

### Example 6: Actinobacillus pleuropneumoniae (App) challenge study in pigs vaccinated with Pasteurella multocida strain PM1094 vaccine

### 6.1 Summary of the study

The protective capacity of strain PM1094 *Pasteurella multocida* bacterine against infection by *Actinobacillus pleuropneumoniae* (App) was evaluated in the present study. Groups of 12 pigs were either vaccinated (group T03) or not (group T04) and later challenge. Vaccination was performed by intramuscular injection, with 2 doses separated by a 2 week interval (D0 and D13) of 1.1 x 1010 CFU of bacteria. Challenge with App was performed by intranasal inoculation of 3.4 x 108 CFU of strain SHOPE 4074 per animal, at D34. Animals were evaluated daily for clinical signs after challenge, with special emphasis on respiratory signs. Vaccinated animals presented a significant increase of antibodies against outer membrane proteins from App. App infected animals (groups T03 and T04) showed signs of acute disease and some animals needed to be euthanatized before the end of the experiment. Although no statistical difference in survival was detected, higher percentage of animals survived in the vaccinated group T03. Lesions associated to App infection were found in both groups T03 and T04. The proportion of affected lung was higher in T04 than in T03, but the difference was not statistically significative. Similarly, re-isolation of App in high quantities was more frequent in the animals from the non-vaccinated group T04 than in the vaccinated group T03. Clinical signs, including fever, were observed in both groups with no statistical differences.

### 6.2 Test product

*Pasteurella multocida* strain PM1094 vaccine
Pharmaceutical form: heat inactivated bacterine, *Pasteurella multocida* strain PM1094 vaccine
Storage conditions: timerosal, 4°C
Supplier: Dept. de Genetica i Microbiologia-UAB
Adjuvant: Aluminium hydroxide (Reheis)
Mock vaccination was performed with PBS and the same adjuvant.

### 6.3 Test System

Animal species: Pigs
Source: A Farm without respiratory clinical signs and historically free of App 1, 9, 11 serotypes.
Number: 24
Sex: The gender of the animals is irrelevant for the objective of the experiment
Age: 3 weeks at the start of the study
Treatments: During the first week, animals were fed with lactofeed containing colistin and zinc. During the following 10 days, a progressive change to starter feed was performed by increasing the proportion of starter feed. Then, only Starter was used to feed the animals until the end of the study. Starter feed contains zinc.
Acclimatization period: After transportation to experimental farm (UAB facilities) and prior to vaccination, the piglets had 17 days of acclimatization

### 6.4 Housing and grouping

Pigs were housed in 2 rooms at UAB farm facilities. Assignment of the piglets to the treatment group was random. Animals were randomly distributed into 2 groups using List Randomizer at www.random.org.

### 6.5 Animal feeding

At the UAB farm, tap water was available ad libitum and feeding was done in accordance to standard UAB procedures. During the first week, animals were fed with lactofeed containing colistin and zinc. During the following 10 days, a progressive change to starter feed was performed by increasing the proportion of starter feed. Then, only Starter was used to feed the animals until the end of the study. Starter feed contains zinc.

### 6.6 Inclusion and exclusion criteria

Animals free of respiratory diseases and free of maternal antibodies against App were selected. Only healthy piglets were included in the study. If the piglets showed any clinical signs not attributable to the vaccination, treatment was to be done. If the treatment did not influence the outcome of the experiment, results were to be included in the evaluation. If during the experiment pigs showed pain or distress, which was considered to be non-transient in nature or likely to become more severe as judged by a veterinarian (in consultation with the study director), they were to be euthanized for animal welfare reasons. If treatment would have influenced the outcome of the results, the animals were to be excluded from the study. Any prophylactic or therapeutic treatments would have been administered only after consultation with the study director.

Twenty four animals arrived to the UAB farm and were distributed in 2 groups of 12 (T03 and T04).

### 6.7 Experimental Design

Twenty four pigs were selected from a farm free of respiratory disease and seronegative to *A. pleuropneumoniae.* Animals were transferred to the farm facilities of the Servei de Granges i Camps Experimentals (SGiCE) from the Universitat Autònoma de Barcelona (UAB) at 3 weeks of age and were allocated in 2 groups of 12 pigs each, which were housed in 2 different rooms. Table 1 summarizes the experimental design.

**Table 2: Summary of experiment**

| Treatment Group | Transport to UAB (3 weeks of age) | Test Product | Day of Admin | Challenge App | Day of euthanasia and necropsy | Number of Animals |
|---|---|---|---|---|---|---|
| T03 | Day -17 | *Pasteurella* vaccine PM1094 | Day 0 and 13 | Day 34 | Day 41 | 12 |
| T04 | | control | | | | 12 |

Vaccination: At 5 (Day 0; D0) and 7 weeks of age (D13), animals in group T03 were vaccinated with the inactivated *P. multocida* strain PM1094vaccine, while animals in group T04 were mock vaccinated with adjuvant (aluminum hydroxide). Serum samples were taken from all the pigs at both days.

Challenge: At 10 weeks of age (D34), animals in groups T03 and T04 were intranasally infected with *A. pleuropneumoniae* strain 4074. Serum samples were collected from all animals at final challenge (D34).

Clinical observation and necroasies: After challenge, animals were observed daily for clinical signs. If signs of suffering were observed, animals were euthanized following protocol SOP PE ML 517. Rectal temperatures were recorded one day before challenge, at challenge and daily after that. Clinical condition observations were performed daily from challenge onwards. Clinical observation included respiratory signs.

One week after *A. pleuropneumoniae* infection all the remaining animals were euthanized and necropsy was performed: Lung lesions were recorded and scored: A lung scoring system (Hannan et al., 1982) and macroscopic examination of pericarditis, pleuritis and lung abscesses presence were carried out. Lung swabs were taken for bacterial culture. PCR was used for confirmation of the results and serum samples were also collected.

### 6.8 Procedures

Vaccination: To test efficacy of vaccines, treatment should be done using the recommended dose and route of vaccination. In this case, vaccinations were performed intramuscularly with 2 ml of the vaccine, equivalent to 2 x 10¹⁰ CFU per animal.
When the piglets were approx. 5 weeks of age (D0), animals from group T03 were vaccinated intramuscularly with 2 ml of the *P. multocida* strain PM 1094 vaccine. Animals in group T04 were mock vaccinated with PBS and adjuvant, aluminum hydroxide. Vaccination was repeated 2 weeks later (D13). Vaccinations were recorded on appropriate forms.

Inoculate for challenge: *Actinobacillus pleuropneumonia* (App), strain SHOPE 4074 (Serotype 1), via: intranasal, at a dose of 3.4 x 10⁸ CFU/animal (in 1.5 ml)

Transport conditions: Bacterial challenge suspensions were kept on ice during transport and were applied at room temperature.

Challenge and preparation of challenge material: Challenge will be done so as to mimic the likely natural route of infection. Groups T03 and T04 will be challenged with App at D35 by intranasal inoculation.

Preparation of App inoculum: App inoculum was prepared from freshly streaked chocolate plates (Chocolate polyvitex agar, Biomerieux). After 5 hours at 37°C and 5% CO₂, the bacterial growth was resuspended in commercial PBS (Lonza, B-4800 Verviers, Belgium) to give an optical density at 660 nm of approximately 0.1 (aprox. 108 CFU/ml). Immediately after the inoculum preparation, the real bacterial concentration was confirmed by dilution and plating on chocolate agar plates and resulted to be 2.3 x 108 CFU/ml. Inoculation was performed intranasally with 1.5 ml of the bacterial suspension (3.4 x 108 CFU per animal) and recorded in the corresponding forms.

Serology: Blood samples were collected individually from all piglets at 1st vaccination (D0) and revaccination (D13), at challenge with App (D34) and at necropsy day (D41/42). Samples were transported to the test laboratory at room temperature. Sera were prepared from the clotted blood samples and were stored at -20°C until used. Blood collection was recorded on appropriate forms. Blood samples taken from the animals included in the study were tested by commercial ELISA kit to detect App ApxIV (REF 99.441188). An ELISA to detect antibodies against OMPs from the vaccine strain, PM1094, and the challenge strain App SHOPE 4074 was also performed by the department of Genetics and Microbiology of the Universitat Autónoma de Barcelona, with serum samples from the animals in groups T03 collected at D0 and D34. In addition, serum samples from 6 animals from T04 collected at D34 were included as control. OMPs were obtained from Pm PM1094 (the vaccine strain), and from App strain used in the challenge. OMPs (0.5 µg per well) were immobilized in microtiter plates and 1/600 serum dilution was used. Polyclonal anti-swine IgG HRP-conjugated antibody (Acris R1384HRP) was employed as secondary antibody. For ELISA assays, the sera of 12 and 7 animals of T3 and T4, respectively, were analyzed.

Clinical condition after challenge: After challenge, all pigs were observed daily for clinical signs of disease. Clinical observation included: nasal discharge, coughing, sneezing, and dyspnea. All observations were recorded in appropriate forms and scored as follows: score: 0 (absence of respiratory signs), score: 1 (mild respiratory signs), score: 2 (moderate respiratory signs), score: 3 (severe respiratory signs).

Other specific signs, if found, were also recorded: Body temperatures of the animals were measured 1 day before, the day of challenge and until the end of the study. For determination of the rectal temperature, a digital thermometer was used. The sensor was placed in the rectum until the number on the display was constant. Evaluation of fever was established by means of a numerical score: 0 (less than 39.5), 1 (between 39.5 and 40.5) and 2 (higher than 40.5). This classification is a modification from that described by Moore et al. (1996). Animals scored 2 were considered to have fever.

Euthanasia, post-mortem examination and sampling: At the end of the experiment, after final bleeding, all animals were euthanized in accordance with standard procedures, using an intravenous overdose of sodium pentobarbital (PE ML 517).

At post-mortem, the animals were examined for lung gross lesions. Particularly, presence of pericarditis, pleuritis and lung consolidation and abscesses were evaluated. Moreover, a lung scoring system according to Hannan et al. (1982) was done. Briefly, each of the 7 lobes of the lungs is allotted a maximum possible lesion score of 5. The area showing pneumonia and /or pleuritis of each lobe is assessed and expressed on a scale of 0-5 to give the pneumonic score per lobe (the maximum total score possible for each complete lung is 35). A tonsilar swab and a lung swab of each lung side were collected from each animal. These samples were transferred to the laboratory where they were immediately processed by means of culture (lung swabs) and PCR (tonsilar swab). Once sampling was completed, all carcasses were destroyed by incineration.

Bacterial culture: Lung swabs were streaked on chocolate agar plates in 2 sections in order to get isolated colonies in the second section. After overnight incubation at 37°C (5% CO2), bacterial growth was recorded and semiquantified in section 1 as: 0, no growth; 1, 1-20 colonies; 2, isolated colonies in a number higher than 20; 3, lawn.

PCR detection: Tonsilar swabs taken at necropsy were tested by PCR to detect App (PT PCT 049), in the animals infected with this bacterium.

### 6.9 Evaluation of results

Primary criteria: presence of lung lesions and percentage of lung area affected. Secondary criteria: clinical respiratory signs, body temperature and mortality associated to App.

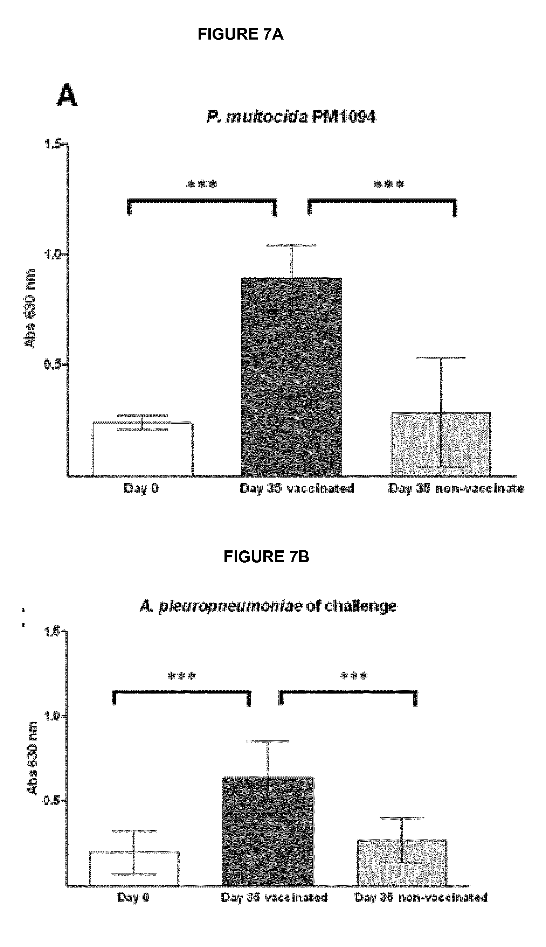

Response to vaccination: All animals treated with the inactivated *Pasteurella multocida* strain PM 1094 vaccine presented a significant increase of the specific IgG against OMP of the App bacteria assayed (Fig 7A and B). In all cases the relative specific IgG amount of the Day 35 of the vaccinated group (T03) was significantly higher than that of the non-vaccinated group (T04) at the same time or the level of antibodies of all the animals at day 0 (Fig. 7A and B).

Presence of lung lesions: Table 3 shows the presence of lesions in animals in groups T03 (vaccinated) and T04 (non-vaccinated). Analysis by X² detected no differences between the two groups in total lesions or in pleuritis and pericarditis.

**Table 3. Number of animals from groups T03 (vaccinated) and T04 (non-vaccinated) with lesions compatible with A. pleuropneumoniae infection.**

| | **Presence of lesions** | | **Presence of pleuritis** | | **Presence of pericarditis** | |
|---|---|---|---|---|---|---|
| | **number** | **%** | **number** | **%** | **number** | **%** |
| **T03** | 9/12 | 75 | 9/12 | 75 | 3/12 | 25 |
| **T04** | 11/12 | 92 | 9/12 | 75 | 5/12 | 42 |

The number of animals with lung lesions in the vaccinated group was lower than in the non-vaccinated group. The origin of the lesions was confirmed by App re-isolation from all cases but from animal 200 from group T04, which had lesions but no positive bacterial isolation. Re-isolation of App was possible from 9 out of 9 animals with lesions in vaccinated animals (T03) and in 10 out of 11 animals with lesions in non-vaccinated animals (T04). The quantity of bacteria found in the lesions was semi-quantified and no differences were found between the isolation from both groups of animals. When the bacterial scores from both samples (left and right lung samples) were added, some differences in the highest score were observed between vaccinated and non-vaccinated animals (Table 3). More animals from the non-vaccinated group showed a score of 6. However, this difference was not statistically significative.

**Table 4. Number of animals from groups T03 (vaccinated) and T04 (non-vaccinated) with re-isolation of A.pleuropneumoniae (App). Quantity of bacteria was semi-quantified in the first section of the agar plate and scored as 0 (no bacteria), 1 (1 to 20 colonies), 2 (isolated colonies in a number higher than 20) and 3 (lawn). The sum of the score from the right and left lung sample was considered in this table.**

| | App re-isolation (score left lung + score right lung) | | | | | | |
|---|---|---|---|---|---|---|---|
| | score 0 | score 1 | score 2 | score 3 | score 4 | score 5 | score 6 |
| T03 | 3/12 (25%) | 0/12 (0%) | 0/12 (0%) | 2/12 (17%) | 1/12 (8%) | 2/12 (17%) | 4/12 (33%) |
| T04 | 2/12 (17%) | 1/12 (8%) | 0/12 (0%) | 1/12 (8%) | 1/12 (8%) | 0/12 (0%) | 7/12 (58%) |

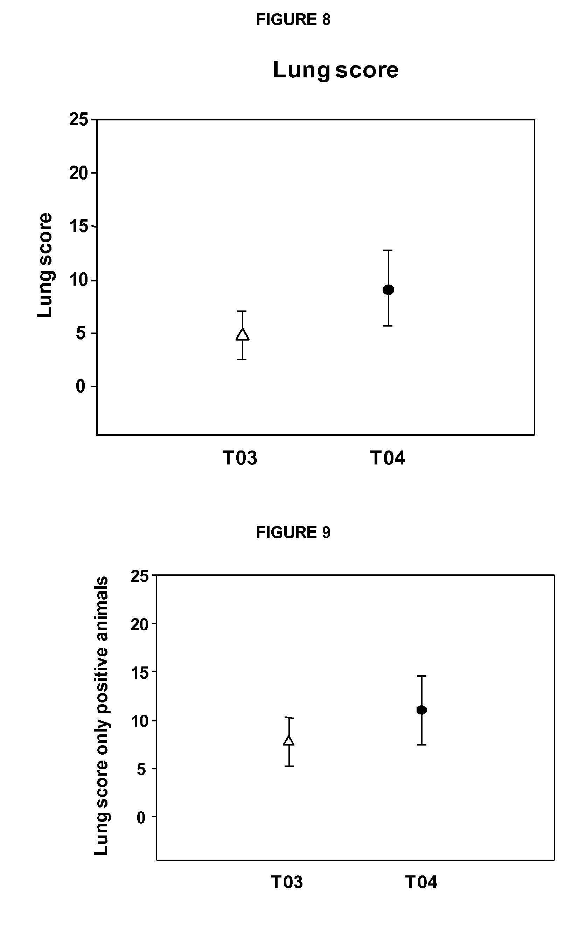

Proportion of lung affected: Lung affected was quantified following a lung scoring system according to Hannan et al. (1982). No statistical difference in lung score was detected when the lung scores from all the animals in groups T03 and T04 were compared (Fig. 8). Although no significant differences were seen, a higher average score was observed in the non-vaccinated animals challenge with App than in the vaccinated ones.

Similar results were obtained when the lung scores from animals with lesions from group T03 and T04 were compared (Fig. 9).

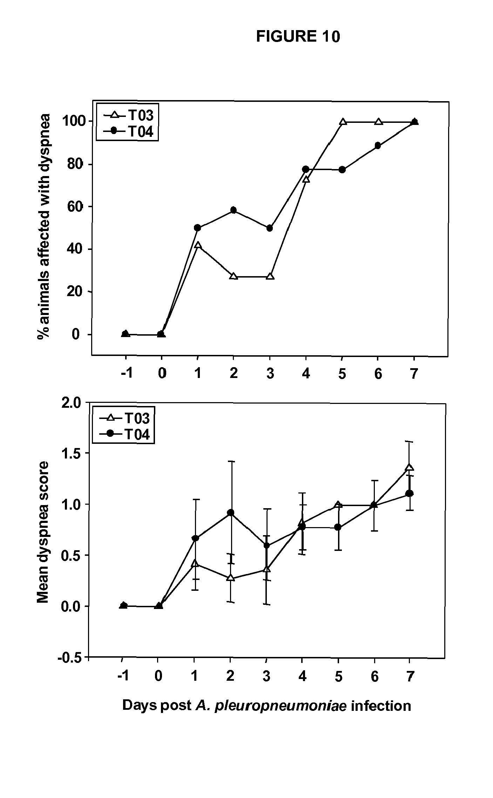

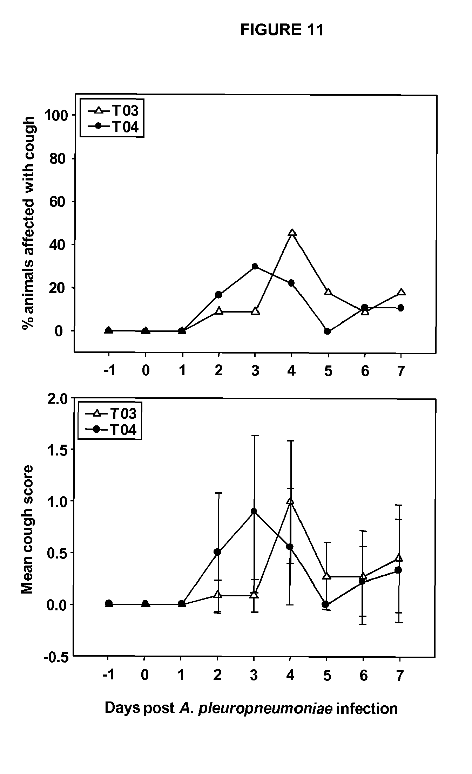

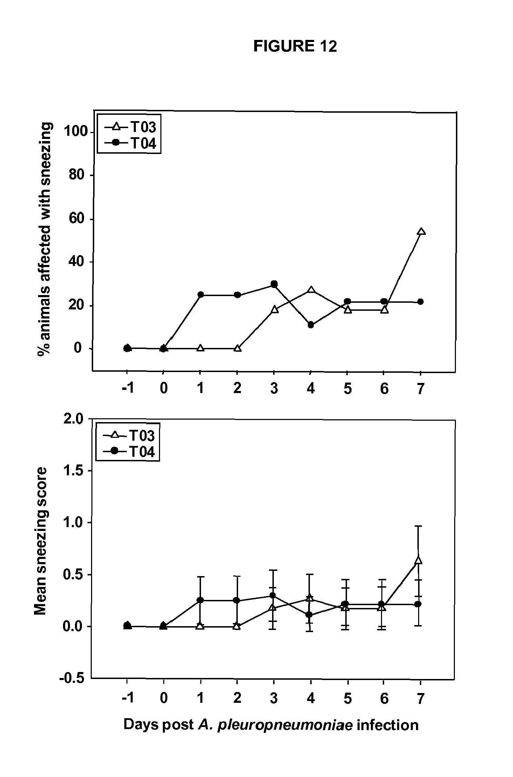

Clinical respiratory signs: Dyspnea, nasal discharge, coughing and sneezing were evaluated after challenge with App. No differences between groups T03 (vaccinated) and T04 (non-vaccinated) were observed in the proportion of animals showing dyspnea (Fig. 10, upper panel) and the severity of this clinical sign (Fig. 10, lower panel) after infection with App. When coughing was analyzed, no significant differences between groups T03 and T04 were detected (Fig. 11). When sneezing was analyzed, a slight delay in the apparition of this respiratory sign was observed in the vaccinated animals, but the differences were not statistically significative (Fisher exact test at 1 and 2 dpi). (Fig. 12)

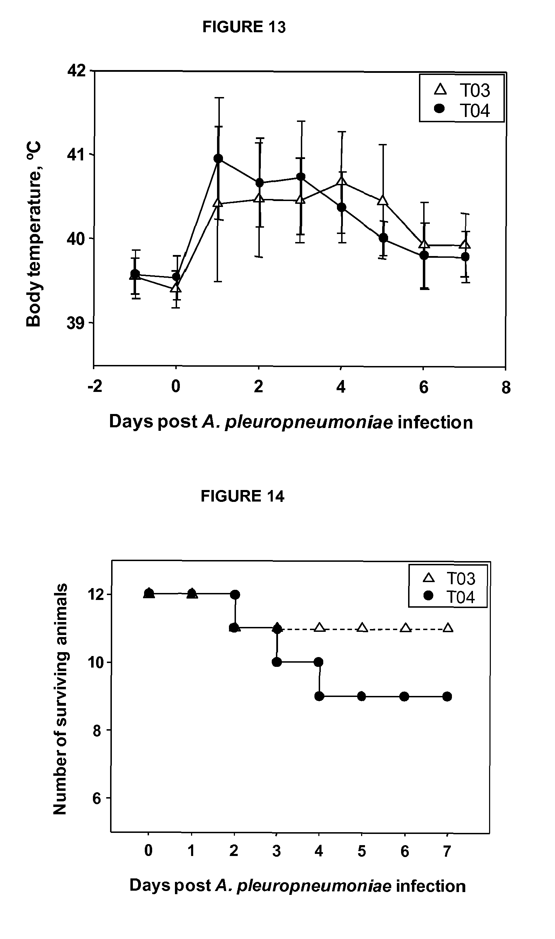

Body temperature: No significant differences were observed in the body temperature of animals from groups T03 (vaccinated) and T04 (non-vaccinated) after infection with App. (Fig.13).

Mortality associated to App infection: As expected, animals infected with App showed mortality associated to the infection (Fig. 14). Mortality started 2 days after inoculation, with 1 animal affected from each group. In group T03 (vaccinated), no more animals needed to be euthanized and 11 animals reached the end of the study. However, in group T04 (control, non-vaccinated), a second animal was euthanized at day 3 and a third animal at day 4 post-inoculation. Nine animals from group T04 reached the end of the study. Survival analysis by Log-Rank or Wilcoxon tests did not detect differences between the two groups. When the X² test was used in the analysis with the animals surviving till the end of the study no differences were detected either between both groups.

### 6.10 Discussion

Vaccination with a heat-inactivated *Pasteurella multocida* (strain PM1094) was effective in eliciting antibodies against the outer membrane proteins (OMP) from the App challenge strain (strain 4074). To improve the cross-reaction of the induced antibodies different means of inactivation of the vaccine, to better maintain the antigenicity of the vaccine strain, or different adjuvants for the vaccine could be explored.

The challenge with App was successful in reproducing disease. App infection produced acute disease and mortality due to the high severity of the disease. More animals from the vaccinated group survived to the end of the study, but survival analysis did not detect significant differences. Lesions due to App infection were detected in both groups of animals, vaccinated or not, with a higher proportion of pericarditis seen in non-vaccinated animals. Re-isolation of App from infected animals was also slightly higher in non-vaccinated animals, with more animals with the maximum score from both lung samples. Accordingly, lung lesion scores were also slightly higher in non-vaccinated animals. Vaccination produced a mild delay in respiratory signs but again, no significant differences were detected in the statistical analysis.

## Claims

1. *Pasteurella multocida* strain PM1094 or an outer-membrane protein extract prepared therefrom for use in the prophylactic and therapeutic treatment of respiratory infections in livestock animals caused by, or associated with at least one bacterial pathogen belonging to the *Pasteurellaceae* family other than any virulent strain and serotype of the bacterial species *Pasteurella multocida.*

2. *Pasteurella multocida* strain PM1094 or an outer-membrane protein extract prepared therefrom for use according to claim 1, wherein the bacterial pathogen belonging to the *Pasteurellaceae* family is selected from the group consisting of: any virulent strain and/or serotype of *Mannheimia sp., Haemophilius sp., Histophilus sp., Pasteurella sp.* (other than *Pasteurella multocida),* and *Actinobacillus sp..*

3. *Pasteurella multocida* strain PM1094 or an outer-membrane protein extract prepared therefrom for use according to any of claims 1 and 2, wherein the bacterial pathogen belonging to the *Pasteurellaceae* family is selected from the group consisting of: any virulent strain and/or serotype of *Manheimia haemolytica, Haemophilus parasuis, Actinobacillus pleuropneumoniae, Histophilus somni, Pasteurella trehalosi* and *Haemophilus paragallinarum.*

4. *Pasteurella multocida* strain PM1094 or an outer-membrane protein extract prepared therefrom for use according to any of claims 1 to 3, wherein the respiratory infections caused by, or associated with at least one pathogen belonging to the *Pasteurellaceae* family are selected from the group consisting of: acute, subacute or chronic pneumonia, shipping fever, pleuropneumonia, porcine respiratory disease complex, bovine respiratory disease complex, upper respiratory tract infections, diphteria, lower respiratory tract infections, Grasser's disease, pasteurellosis.

5. *Pasteurella multocida* strain PM1094 or an outer-membrane protein extract prepared therefrom for use according to any of claims 1 to 4, in the prophylactic and therapeutic treatment of respiratory infections in livestock animals selected from the group consisting of: cattle, horses, pigs, birds, poultry, sheep, goat and rabbit.

6. *Pasteurella multocida* strain PM1094 or an outer-membrane protein extract prepared therefrom for use according to any of claims 1 to 5 in the prophylactic and therapeutic treatment of respiratory infections in pigs caused by or associated with *Actinobacillus pleuropneumoniae.*

7. *Pasteurella multocida* strain PM1094 or an outer-membrane protein extract prepared therefrom for use according to any of claims 1 to 5 in the prophylactic and therapeutic treatment of respiratory infections in pig, cattle or sheep caused by or associated with *Manheimia haemolytica.*

8. *Pasteurella multocida* strain PM1094 or an outer-membrane protein extract prepared therefrom for use according to any of claims 1 to 5 in the prophylactic and therapeutic treatment of respiratory infections in pigs caused by or associated with *Haemophilus parasuis.*

9. *Pasteurella multocida* strain PM1094 or an outer-membrane protein extract prepared therefrom for use according to any of claims 1 to 5 in the prophylactic and therapeutic treatment of respiratory infections in cattle caused by or associated with *Histophilus somni.*

10. *Pasteurella multocida* strain PM1094 or an outer-membrane protein extract prepared therefrom for use according to any of claims 1 to 5 in the prophylactic and therapeutic treatment of respiratory infections in sheep caused by or associated with *Pasteurella trehalosi.*

11. *Pasteurella multocida* strain PM1094 or an outer-membrane protein extract prepared therefrom for use according to any of claims 1 to 5 in the prophylactic and therapeutic treatment of respiratory infections in poultry caused by or associated with *Haemophilus paragallinarum.*

12. A vaccine composition against respiratory infections in livestock animals caused by, or associated with at least one bacterial pathogen belonging to the *Pasteurellaceae* family other than any virulent strain and serotype of the bacterial species *Pasteurella multocida* comprising an immunogenically effective amount of *Pasteurella multocida* strain PM1094 or an outer-membrane protein extract prepared therefrom together with an acceptable carrier, vehicle and/or adjuvant.
